(19) Europäisches Patentamt

European Patent Office

Office européen des brevets

(11) EP 1 395 647 B1

(12) **EUROPEAN PATENT SPECIFICATION**

(45) Date of publication and mention
of the grant of the patent:
**22.11.2006 Bulletin 2006/47**

(21) Application number: **01992263.2**

(22) Date of filing: **18.12.2001**

(51) Int Cl.:
*C12M 1/36* *(2006.01)*        *C12M 3/02* *(2006.01)*

(86) International application number:
**PCT/US2001/049761**

(87) International publication number:
**WO 2002/050251 (27.06.2002 Gazette 2002/26)**

(54) **A DEVICE AND METHOD FOR SEED-TRAIN EXPANSION OF MAMMALIAN CELLS**

VORRICHTUNG UND VERFAHREN ZUR SERIENVERMEHRUNG VON SÄUGERZELLEN ZUM ANIMPFEN

DISPOSITIF ET PROCEDE D'EXPANSION DE TRAINS DE SEMENCES POUR DES CELLULES DE MAMMIFERES

(84) Designated Contracting States:
**DE ES FR GB IT**

(30) Priority: **20.12.2000 US 746972**

(43) Date of publication of application:
**10.03.2004 Bulletin 2004/11**

(73) Proprietor: **Bayer Pharmaceuticals Corporation West Haven, CT 06516 (US)**

(72) Inventors:
- **HEIDMANN, Rudiger**
Emeryville, CA 94608 (US)
- **MERED, Mokhtar**
Orinda, CA 94563 (US)
- **MICHAELS, James, D.**
Oakland, CA 94610 (US)
- **KONSTANTINOV, Konstantin**
Walnut, CA 94596 (US)

(74) Representative: **Burkert, Frank**
**Bayer HealthCare AG**
**CAO Law and Patents**
**Patents and Licensing**
**51368 Leverkusen (DE)**

(56) References cited:
**EP-A- 0 348 827**        **EP-A- 0 487 867**
**DE-A- 4 324 293**        **US-A- 3 591 460**

- **HEIDEMANN RUDIGER ET AL: "The one step inoculation concept: A new seed-train expansion for recombinant mammalian cell lines." ABSTRACTS OF PAPERS AMERICAN CHEMICAL SOCIETY, vol. 221, no. 1-2, 1 April 2001 (2001-04-01), page BIOT 199, XP008022337 221st National Meeting of the American Chemical Society;San Diego, California, USA; April 01-05, 2001 ISSN: 0065-7727**

## Description

BACKGROUND OF THE INVENTION

Field: This invention relates to manufacturing scale mammalian cell culture technology and specifically to a new seed-train expansion method for mammalian cells. Cryopreserved cells are used to inoculate directly into a newly designed bioreactor, which serves as a seed source for production scale.

Background: The start of a new cell seed-train expansion for a production campaign for the expression of a mammalian cell culture system is a critical process step. Operational inconsistencies and errors often compromise the process, leading to significant delays and inoculum variability. In typical production protocols, a new seed train expansion begins from a 1-2 mL cryovial (master working cell bank, MWCB). The cell concentration of this container is usually in the range of 5 - 10 million cells/mL. The cells are thawed, washed to remove the cryoprotectant and then first cultured (seeded) in small tissue culture flasks. It is standard practice with mammalian cell cultures to use inoculation densities of 0.5 to 1 x $10^6$ cells/mL. Seeding cells at lower cell concentrations after cryopreservation may result in an extended lag phase prior to entering the growth phase, poor cell performance or even cell death. This is especially accentuated in the serum-free or even protein-free cell culture media that has become the modus operandi for current cell culture manufacturing.

The cells are sub-cultivated according to their specific growth rate (cell density), and are usually split into multiple cell culture flasks every 2 - 3 days. Once enough biomass is produced, the cells are expanded into larger cultivation bottles such as roller bottles, shake or spinner flasks. After enough cell mass is accumulated, a bioreactor, which becomes the seed culture for the production-scale vessel, is inoculated. This described seed-train expansion is general practice for several mammalian cell lines and is widely used in commercial production and academia. An overview of a commercial seed train expansion using T-flasks and spinner flasks is also given by *Whitaker et al., Journal of the American Chemical Society, pages 28-43, 1998.*

Typical scale-up protocols which take about four to six weeks to complete under optimal conditions are labor intensive and are susceptible to contamination and variability. The small scale conditions are not well defined - usually there is no set-point for pH and DO during the T-flask, roller bottle or shaker flask period, which may lead to more variability throughout the process and might compromise the quality of the inoculum and the product.

SUMMARY OF THE INVENTION

We have developed a novel bioreactor and method for the seed-train expansion of mammalian cells.

This technology eliminates the use of tissue culture-flasks, roller bottles or shake flasks.

Our novel inoculation bioreactor is designed to facilitate an improved method of mammalian cell seed-train expansion, and is distinguished by the presence of an "inoculation well" which communicates with the interior of the bioreactor and which facilitates the growth of mammalian cells for commercial seed-train expansion.

Our method for seed-train expansion of cryopreserved cells comprises the use of a dedicated inoculation bioreactor having an inoculation well for expanding the cells prior to their transfer to a production bioreactor. More specifically, our method comprises adding the cryopreserved cells to media within the inoculation well of the inoculation bioreactor, enabling the cells to grow to a predetermined concentration within the inoculation well by monitoring and adjusting the condition of the media and environment, and thereafter incrementally increasing the volume of the media within the reactor so that optimal cell growth is maintained. Once the desired cell density and volume is achieved, the cells are transferred to a production bioreactor.

In a preferred embodiment, the inoculation bioreactor includes an "inoculation well" located at the base of the reactor chamber. This inoculation well is supplied with fermentation sensors (pH, dissolved oxygen, temperature, optical density, etc.) to assist in assuring optimal cultivation conditions. Most preferably, this inoculation well is adapted to include an impeller drive by a continuously stirred tank reactor (CSTR) liquid agitation device.

BRIEF DESCRIPTION OF THE FIGURES

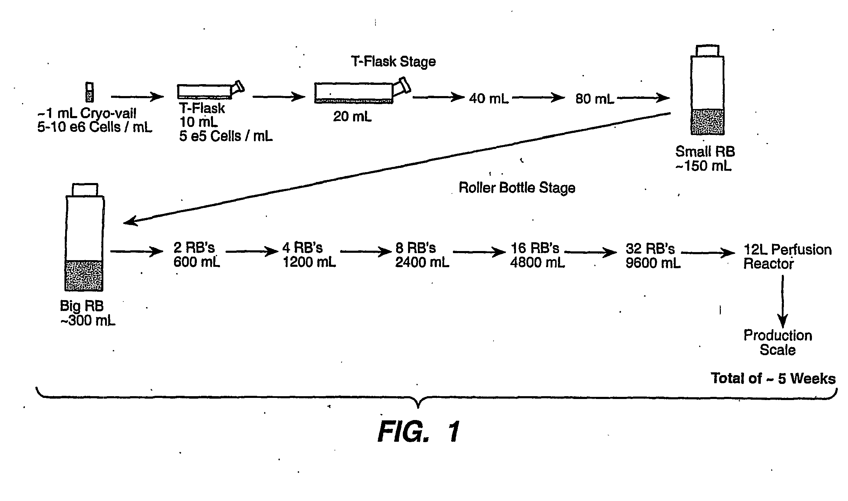

**Figure 1** illustrates the prior art of seed-train expansion method.

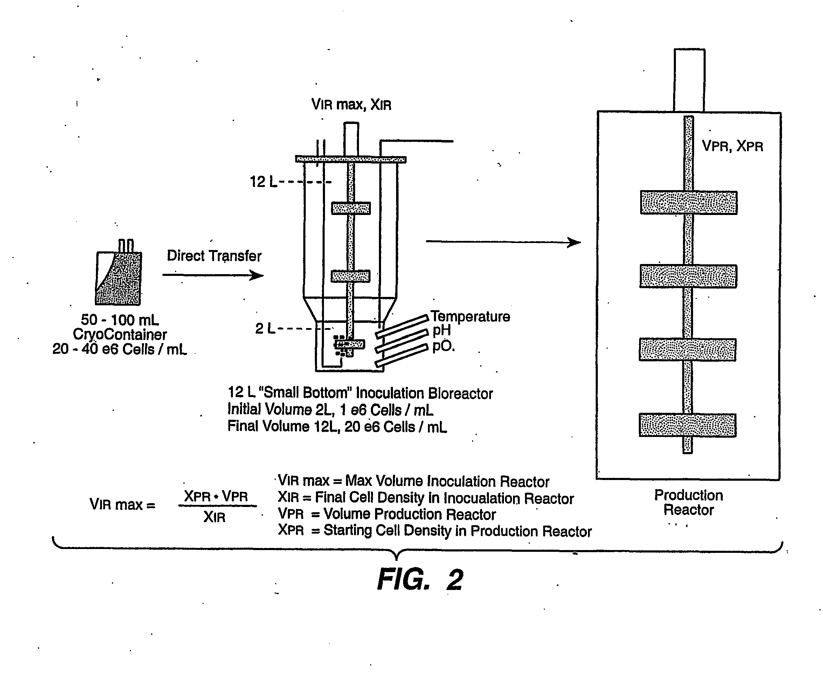

**Figure 2** shows the preferred method of this invention.

**Figure 3** shows the concept of the inoculation well inoculation bioreactor.

**Figure 4** shows a continuous perfusion culture performed in the 5 L "inoculation well" containing bioreactor, started with BHK cells cryopreserved in the 50 mL bag. The viable cell density (VCD [•]) and the viability profile [ ] are shown. Cell viability remained high throughout the culture. The target cell density of 20 million cells/mL at 5 L scale was reached within 12 days.

**Figure 5** illustrates the influence of a DMSO washing step during the seed-train expansion of BHK cells. In the 12 L culture (open symbols) a DMSO washing step was performed were as the second 5 L culture (filled symbols) was directly inoculated without washing the cells. As indicated no negative effect was observed, cell viability as well as cell growth showed no difference

**Figure 6** shows a continuous 12 L bioreactor culture, started with BHK cells cryopreserved in the 50 mL bag, no DMSO wash was performed before the inoculation. The viable cell density (VCD [•]) and the viability profile [ ] are shown. The target cell density of 20 million cells/mL at 12 L scale was reached within 14 days.

**Figure 7** illustrates a 5 L culture of recombinant CHO cells, started with CHO cells cryopreserved in a 50 mL bag, no DMSO wash was performed before the inoculation. The viable cell density (VCD [•]) and the viability profile [ ] are shown. The target cell density was reached within 6 days.

**Figure 8a** illustrates two 5 L cultures started with rBHK cells cryopreserved in a 50 mL bag, a second culture in a 100 mL bag, no DMSO wash was performed before the inoculation. The viable cell density (VCD [•]) and the viability profile [ ] are shown. The target cell densities of 20 million cells/mL at 5 L scale was reached within 10 days in both cultures.

**Figure 8b** illustrates two 12 L cultures started with recombinant CHO cells cryopreserved in 50 mL and 100 mL bags, no DMSO wash was performed before the inoculation. The viable cell density (VCD [•]) and the viability profile [ ] are shown. No negative effect was noticed in both cultures.

SPECIFIC EMBODIMENTS

Materials and Methods

This seed-train expansion method was initially developed for a recombinant baby hamster kidney (BHK) cell line and a chinese hamster ovary (CHO) cell line expressing human proteins. Cells used for this invention were taken from 12 L perfusion bioreactor containing 20 x $10^6$ cells/mL. The cells were cultivated and frozen in mammalian cell culture medium based on a commercially available DMEM/Ham's F12 formulation (1:1) manufactured by JRH (Lenexa, KS) or Life Technologies (Grand Island, NY) and supplemented with iron, Pluronic F68 (BASF, Pardipanny, NJ), recombinant human insulin (Humulin, Eli Lilly, Indianpolis, IN), and essentially free of other proteins. The freezing medium contained 7.5% dimethylsulfoxide (Sigma, St. Louis, MO) as a cryoprotectant.
The cryopreservation bags (Cryocyte™ 250 mL or 500 mL, Nexell Therapeutics Inc. Irvine, CA) used for storage contained 50 - 100 mL of cell suspension and were frozen in a -40°C freezer (Revco, Asheville, NC) prior to transferring to a liquid $N_2$ freezer (Forma Scientific, OH). The cells were concentrated to approx. 40 x $10^6$ cells/mL in 50 mL, or to 20 x $10^6$ cells/mL in 100 mL to ensure an initial bioreactor cell density of 1 million cell/mL in 2 L volume. Cell densities of 0.5 - 1 $10^6$ cells/mL are commonly used to initiate a new seed-train expansion process.
The BHK cells in the cryopreservation bag were thawed using a 37°C water bath. The cell suspension was transferred to a centrifuge tube, diluted with fresh medium (1:1) and gently centrifuged at 1000 - 1200 rpm. The supernatant was discarded, the cell pellet resuspended in fresh medium and transferred to a sterile bottle to inoculate the "inoculation well" bioreactor. This wash step is used to remove most of the DMSO and is common practice in mammalian cell culture. Later, this DMSO washing step was eliminated and the cells were directly transferred into the bioreactor from the bag. (Elimination of the DMSO wash step allows operation and maintenance of a closed system, thereby reducing the opportunity for system contamination. See Example 2.)
The same technology was also used for recombinant CHO-cells.
Two different bioreactors were used. However, both of them had the common feature of being able to inoculate into a 2 L volume "inoculation well".

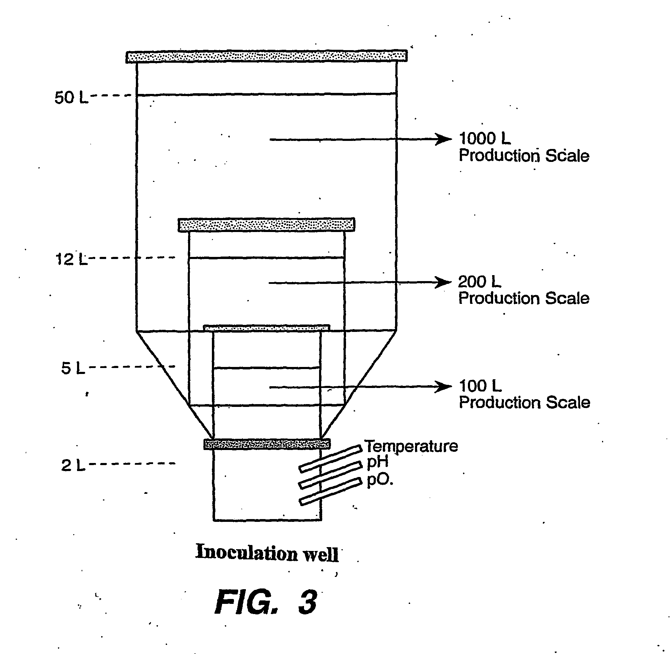

1. 5 L cultures were carried out in Applikon 7 L bioreactors (Schiedam, The Netherlands) equipped with a sintered stainless steel frit (Mott Metallurgical, Farmington, CT) for aeration. A cell separation device was used to retain cells within the vessel and to allow for operation in continuous perfusion mode. The reactor system is capable of supporting at least 20 million cells/mL in 5 L volume. pH, dissolved oxygen, temperature, and optical density were measured via probes that were submerged in culture when a volume of at least 2 L was used. The reactor is designed with a 2 L volume stainless steel inoculation well.
2. 12 L cultures were carried out in custom designed 15 L bioreactor. The stainless steel inoculation well of the reactor was the same (2 L volume) as in the 7 L vessel. However, a conical flange was used to extend the diameter and the volume of the top part (figure 3). Aeration was provided through a sintered stainless steel frit (Mott Metallurgical, Farmington, CT). A cell separation device was used for continuous perfusion technology. This reactor system is capable of supporting at least 20 million cells/mL in a 12 L working volume.

Both bioreactor systems were controlled using a B. Braun DCU (Digital Control Unit, B. Braun International, Melsungen, Germany) under operating conditions as reported elsewhere.
The relationship between the size and cell density of the used cryo-container and the inoculation well

($V_{IR\ min}$) of the inoculation reactor can be determined by following formula:

$$V_{IR\ min} = \frac{X_{bag} * V_{bag}}{X_{IR\ start}}$$

were $X_{bag}$ is the cell density in the cryo-container and $V_{bag}$ the volume. $X_{IR\ start}$ is the desired starting cell density in the inoculation well of the inoculation reactor, usually 1 x $10^6$ cells/mL.

The final volume of the inoculation reactor ($V_{IR\ max}$) depends on the volume of the production reactor that will be used and can be determined by the formula:

$$V_{IR\ max} = \frac{X_{PR} * V_{PR}}{X_{IR}}$$

where $X_{PR}$ is the targeted initial cell density in the production bioreactor (usually 1 $10^6$ cells/mL), $V_{PR}$ the volume of the production reactor, and $X_{IR}$ the final cell density in the inoculum bioreactor. The inoculation well of the bioreactor will normally be small (in the present examples a 2L inoculation well was used) and the volume may be increased by increasing the diameter and the height of the bioreactor. Since $V_{IR\ max}$ is not limiting, the same "inoculation well" concept can be applied to the design of larger bioreactors. (see figure 3).

The cultures carried out in the 7 and 12 L systems had an initial volume of 2 L. This is the minimum volume necessary to submerge the bioreactor's pH, dissolved oxygen electrodes, as well as the temperature sensor. Aeration is carried out using headspace aeration just after inoculation. The volume is increased stepwise to keep the cell density between 0.8 and 1.2 x $10^6$ cells/mL. Oxygenation via gas sparging is used once the volume reaches four liters. The cell-specific perfusion rate was maintained at 0.5 - 0.7 nL/cell/day at all times.

Off-line sampling was performed daily to determine cell and metabolite concentrations. Cell counts and viability were determined using a hemacytometer and the trypan blue exclusion method. A YSI analyzer (Yellow Springs Instruments) was used to measure glucose, lactate, glutamate and glutamine concentrations of samples. LDH and ammonia were measured using a Kodak Biolyzer (Kodak Instruments, NY). A NOVA blood gas analyzer (Nova Biomedical, Waltham, Mass.) was used to measure the dissolved $CO_2$ level and to check pH and dissolved oxygen values. Samples were analyzed for rFVIII activity by the one stage coagulation method. The product quality was determined by an in-house developed Western Blot assay.

EXAMPLE 1

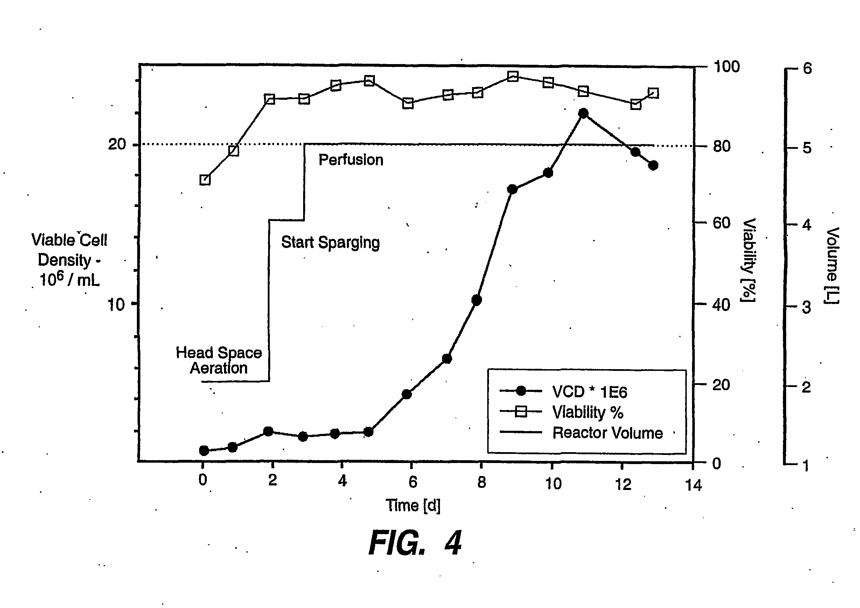

Figure 4 shows a 12 L perfusion culture. 50 mL of cell suspension cryopreserved in an EVA bag was used for inoculation. The cells were washed with fresh medium to remove the DMSO before inoculation. The culture was at an initial volume of 2 L with headspace aeration. The cell viability exceeded 95% throughout the process and the volume was increased stepwise based on cell density. Gas sparging was used once the volume was at 4 L. Continuous medium perfusion was started one day after the volume reached 12 L. The perfusion rate was kept at 0.5 - 0.7 nL/cell/day. The target cell density of 20 $10^6$ cells/mL was reached after 11 days. The seed-train expansion experiment clearly demonstrates the elegance of this new expansion technology. No intermediate cell culture flasks were used, the risk of possible contamination was dramatically reduced, the media and environment were externally controlled, and as a result the time to start a production bioreactor was reduced by at least 70%.

EXAMPLE 2

A necessity of the DMSO removal step (wash/dilution) before the inoculation was evaluated. In this evaluation, two reactors were started in parallel from the same 50 mL bag freeze lot.

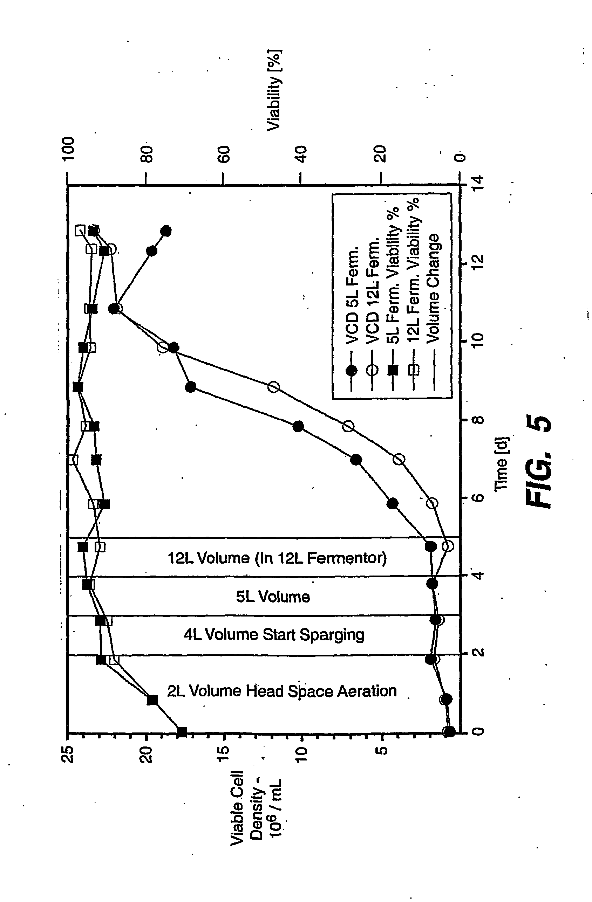

• 12 L reactor where a DMSO washing step was performed.
• 5 L reactor where a DMSO wash was omitted.
  Both cultures were run under the same conditions (volume increase steps, headspace aeration and sparging rates etc.). The 5 L culture reached the target cell density of 20 $10^6$ cells/mL within 11 days, the 12 L culture one day later. Figure 5 shows a comparison of these two cultures. No difference in cell growth or viability was noticed. Elimination of the DMSO wash step further streamlines the seed-train expansion process (from cell thaw onwards) and allows for system closure throughout.

EXAMPLE 3

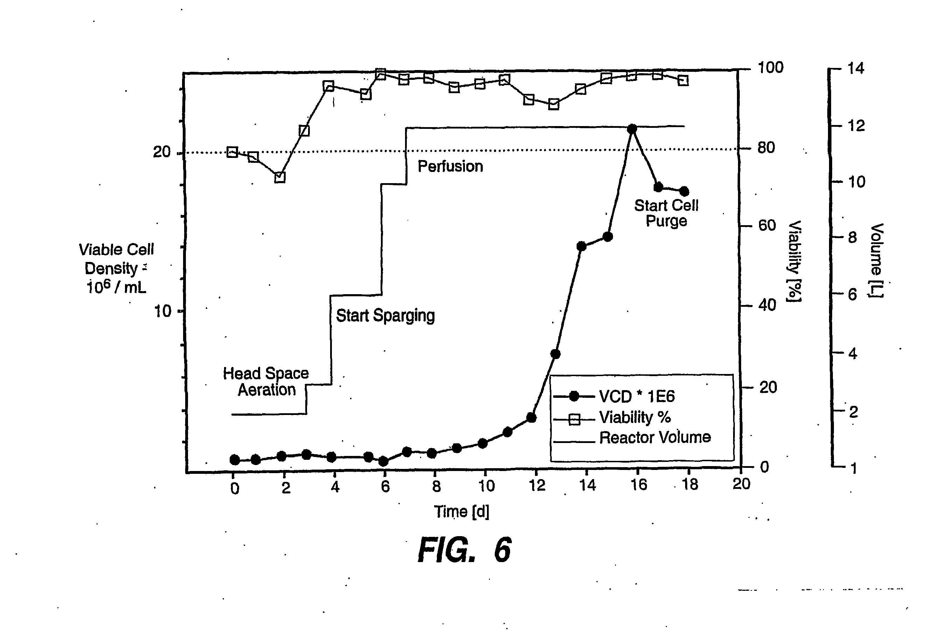

Figure 6 shows the time profile of a culture performed in the 12 L "inoculation well" reactor. 50 mL of cell suspension, cryopreseved in a bag, was used for inoculation without a DMSO wash. The target cell density of 20 x $10^6$ cells/mL was reached after 16 days.

EXAMPLE 4

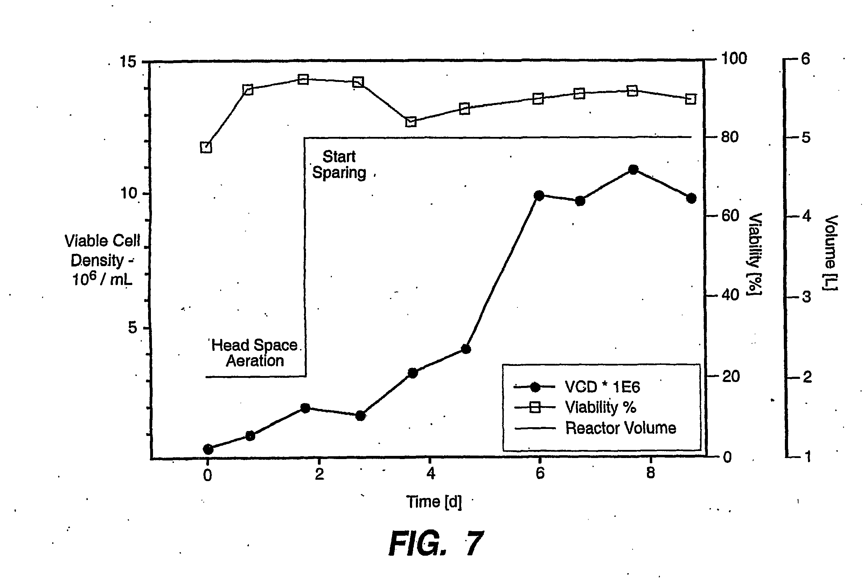

Figure 7 shows the time profile of a CHO culture performed in the 5 L bioreactor. 50 mL of cryopreseved cells in a bag was used for inoculation. No DMSO washing step was performed. The target cell density - here 10 x $10^6$ cells/mL were reached within 6 days.

EXAMPLE 5

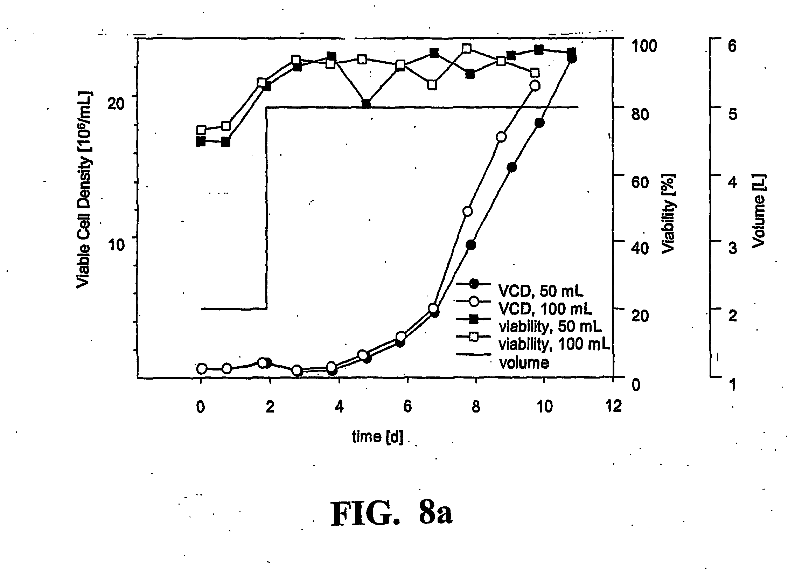

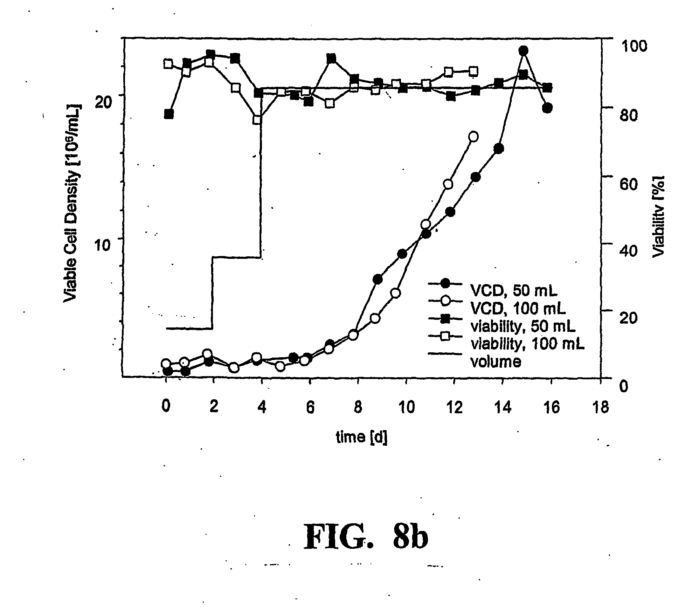

Figure 8a and 8b show the time profiles of two BHK and two CHO cultures performed in the inoculation bioteactors. 50 and 100 mL cryobags were used for inoculation, no DMSO washing step was performed in all four cultures. No negative effect was noticed using either 50 mL or 100 mL cryobags.

SUMMARY

In general, existing seed-train expansion methods for mammalian cell culture are labor intensive and susceptible to microbial contamination due to the use of multiple cultivation vessels such as T-flasks, roller-bottles and spinner flasks. A new seed-train expansion method was developed utilizing 50 mL or 100 mL EVA cryopreservation bags that are used to inoculate directly into a custom built "inoculation well" in a bioreactor. This reactor serves as the inoculation source for a production scale system. This methodology completely eliminates the use of any T-flasks, roller bottles or spinner flasks during the scale-up. A procedure for sizing and design of the "inoculation well" reactor is proposed. Following this procedure, the method can be applied for the direct inoculation of any scale production reactor. Cryopreserved cells were used in these examples, but cryopreservation is not a requirement of this technology. Mammalian cells at standard temperatures can be substituted for the cryopreserved cells and the effectiveness and utility of the system would remain the same. Similarly, the size of the bioreactor can be adjusted, so that as long as the concepts of an inoculation well and the method of use are maintained.

**Claims**

1. A method of mammalian cell seed-train expansion comprising:

   a) providing a bioreactor having an inoculation well,
   b) delivering a suspension of mammalian cells to media within the inoculation well,
   c) controlling environmental conditions and composition of said media so that cell growth within the inoculation well is optimized,
   d) growing the mammalian cells until a predetermined cell density is reached within said well, and
   e) increasing the media volume incrementally while maintain optimum environmental conditions and environmental growth conditions until the bioreactor is filled to a predetermined volume and cell density.

2. The method of claim 1, wherein the mammalian cells are cryopreserved cells.

3. The method of Claim 1, wherein the mammalian cells are selected from the group consisting of chinese hamster ovary cells and baby hamster kidney cells.

4. The method of claim 1, wherein the cells are obtained from a cryobag.

5. The method of claim 1, wherein the cells are obtained from a cryovial.

6. A fermentation bioreactor comprising:

   a) a bioreactor, said bioreactor defining a chamber,
   b) said chamber having exterior and interior sides, a top member, wall member, and base member, said base member having an orifice,
   c) an inoculation well, said well defining a chamber having exterior and interior sides, top and base members, a wall member, and
   d) said well further including an orifice in said top member joined to said bioreactor orifice, so that a channel is formed between the well chamber and the bioreactor chamber to permit unrestricted movement there between.
   e) said well chamber being provided with an optical sensor

7. A bioreactor as recited in Claim 6, in which the inoculation well chamber is provided with sensors selected from the group consisting of: pH sensor, oxygen sensor, temperature sensor.

8. A bioreactor as recited in Claim 6, in which the inoculation well is provided with a communication orifice through the wall of said well to permit the introduction of media and agents into the well chamber.

9. A bioreactor as recited in Claim 6, in which the inoculation well is provided with a mechanical arm to permit stirring of the contents of said well.

**Patentansprüche**

1. Verfahren zur Säugetierzellenimpfgut-Folgevermehrung ("seed-train expansion"), umfassend:

   a) das Bereitstellen eines Bioreaktors mit einem Inokulationswell,
   b) das Zuführen einer Suspension von Säugetierzellen zu einem Medium im Inokulationswell,
   c) das Kontrollieren der Umgebungsbedingungen und der Zusammensetzung des Mediums, sodass das Zellwachstum im Inokulationswell optimiert wird,

d) das Züchten der Säugetierzellen, bis eine vorbestimmte Zelldichte im Well erreicht ist, und

e) das schrittweise Erhöhen des Medienvolumens, während die optimalen Umgebungsbedingungen und Umgebungswachstumsbedingungen aufrechterhalten werden, bis der Bioreaktor mit einem vorbestimmten Volumen und einer vorbestimmten Zelldichte gefüllt ist.

**2.** Verfahren nach Anspruch 1, worin die Säugetierzellen kryokonservierte Zellen sind.

**3.** Verfahren nach Anspruch 1, worin die Säugetierzellen aus der aus Chinahamster-Ovarialzellen und Babyhamster-Nierenzellen bestehenden Gruppe ausgewählt sind.

**4.** Verfahren nach Anspruch 1, worin die Zellen aus einem Kryobeutel stammen.

**5.** Verfahren nach Anspruch 1, worin die Zellen aus einem Kryoröhrchen stammen.

**6.** Fermentationsbioreaktor, umfassend:

a) einen Bioreaktor, wobei der Bioreaktor eine Kammer definiert,

b) wobei die Kammer eine Außen- und eine Innenseite, ein Deckelement, ein Wandelement und ein Basiselement aufweist, wobei das Basiselement eine Öffnung aufweist,

c) einen Inokulationswell, wobei der Well eine Kammer mit einer Außen- und einer Innenseite, einem Deck- und Basiselement und einem Wandelement definiert und

d) wobei der Well weiters eine Öffnung im Deckelement aufweist, die mit der Bioreaktoröffnung verbunden ist, sodass ein Kanal zwischen der Wellkammer und der Bioreaktorkammer gebildet wird, der eine uneingeschränkte Bewegung dazwischen erlaubt,

e) wobei die Wellkammer mit einem optischen Sensor ausgestattet ist.

**7.** Bioreaktor nach Anspruch 6, worin die Inokulationswellkammer mit Sensoren ausgestattet ist, die aus der aus pH-Sensor, Sauerstoffsensor, Temperatursensor bestehenden Gruppe ausgewählt sind.

**8.** Bioreaktor nach Anspruch 6, worin der Inokulationswell mit einer Kommunikationsöffnung durch die Wand des Wells ausgestattet ist, um die Einführung eines Mediums und von verschiedenen Agenzien in die Wellkammer zu ermöglichen.

**9.** Bioreaktor nach Anspruch 6, worin der Inokulationswell mit einem mechanischen Arm ausgestattet ist, um das Rühren des Inhalts des Wells zu ermöglichen.

**Revendications**

**1.** Procédé d'amplification de chaînes d'ensemencement de cellules de mammifère, consistant :

a) à prendre un bioréacteur ayant un puits d'inoculation,

b) à faire arriver une suspension de cellules de mammifère à des milieux contenus dans le puits d'inoculation,

c) à régler les conditions environnementales et la composition des milieux en question de manière à optimiser la croissance des cellules dans le puits d'inoculation,

d) à faire croître les cellules de mammifère jusqu'à ce qu'une densité cellulaire prédéterminée soit atteinte dans le puits, et

e) à accroître le volume des milieux par paliers tout en entretenant des conditions optimales d'environnement et des conditions optimales de croissance environnementale jusqu'à ce que le bioréacteur soit rempli jusqu'à un volume et une densité cellulaire prédéterminés.

**2.** Procédé suivant la revendication 1, dans lequel les cellules de mammifère sont des cellules cryoconservées.

**3.** Procédé suivant la revendication 1, dans lequel les cellules de mammifère sont choisies dans le groupe consistant en cellules ovariennes de hamsters chinois et cellules rénales de jeunes hamsters.

**4.** Procédé suivant la revendication 1, dans lequel les cellules proviennent d'une poche de cryoconservation.

**5.** Procédé suivant la revendication 1, dans lequel les cellules proviennent d'une fiole de cryoconservation.

**6.** Bioréacteur de fermentation, comprenant :

a) un bioréacteur qui définit une chambre,

b) la chambre en question ayant des côtés extérieur et intérieur, un élément supérieur, un élément de paroi et un élément inférieur, cet élément inférieur présentant un orifice,

c) un puits d'inoculation, ce puits définissant une chambre ayant des côtés extérieur et intérieur, des éléments supérieur et inférieur et un élément de paroi, et

d) ce puits présentant en outre un orifice ménagé dans l'élément supérieur relié à l'orifice du bioréacteur, de manière à former un canal entre la chambre du puits et la chambre du bioréacteur pour permettre entre elles un mouvement libre, e) la chambre du puits étant pourvue d'un capteur optique.

7.  Bioréacteur suivant la revendication 6, dans lequel la chambre du puits d'inoculation est pourvue de capteurs choisis dans le groupe comprenant : un capteur de pH, un capteur d'oxygène, un capteur de température.

8.  Bioréacteur suivant la revendication 6, dans lequel le puits d'inoculation présente à travers sa paroi un orifice de communication permettant l'introduction de milieux et d'agents dans la chambre du puits.

9.  Bioréacteur suivant la revendication 6, dans lequel le puits d'inoculation est équipé d'un bras mécanique permettant d'agiter le contenu du puits.

FIG. 1

EP 1 395 647 B1

VIR max, XIR

VPR, XPR

12 L

2 L

Direct Transfer

Temperature
pH
pO.

50 - 100 mL
CryoContainer
20 - 40 e6 Cells / mL

12 L "Small Bottom" Inoculation Bioreactor
Initial Volume 2L, 1 e6 Cells / mL
Final Volume 12L, 20 e6 Cells / mL

$$VIR\ max = \frac{XPR \cdot VPR}{XIR}$$

VIR max = Max Volume Inoculation Reactor
XIR = Final Cell Density in Inocualation Reactor
VPR = Volume Production Reactor
XPR = Starting Cell Density in Production Reactor

Production
Reactor

## FIG. 2

EP 1 395 647 B1

Inoculation well

*FIG. 3*

FIG. 4

FIG. 5

FIG. 6

FIG. 7

FIG. 8a

FIG. 8b